# EUROPEAN PATENT APPLICATION

(11) **EP 2 561 865 A2**
(43) Date of publication of application: **27.02.2013**
(21) Application number: 11772254.6
(22) Date of filing: 21.04.2011
(51) Int. Cl.: A61K 9/70, A61K 31/404, A61P 25/00

(54) **TRANSDERMAL ABSORPTION PREPARATION**

(30) Priority: 23.04.2010 KR 20100038184
(71) Applicant: Icure Pharmaceutical Inc., Seoul 151-015 (KR)
(72) Inventor: CHOI, Young Kweon, Seoul 135-280 (KR); KIM, Jung Ju, Seoul 135-828 (KR); KIM, Seong Su, Seongnam-si, Gyeonggi-do 462-839 (KR); LEE, Jung Sik, Suwon-si, Gyeonggi-do 443-374 (KR); PARK, Seong Min, Seoul 157-010 (KR)
(74) Representative: Michalski, Stefan
(86) International application number: PCT/KR2011/002887
(87) International publication number: WO 2011/132966

(57) **Abstract**

The present invention relates to a transdermal absorption preparation. The transdermal absorption preparation of the present invention comprises a drug-containing adhesive layer, a drug-protective layer and a release layer, wherein the drug-containing adhesive layer contains ropinirole or a salt thereof, rubber, an adhesion-imparting resin, an antioxidant, and a transdermal absorption promoter. Also, the present invention provides a transdermal absorption preparation comprising a drug-containing adhesive layer, a drug-protective layer, and a release layer, wherein the drug-containing adhesive layer contains ropinirole or a salt thereof, acrylic rubber, an anti-crystallization agent, and a transdermal absorption promoter. When the transdermal absorption preparation of the present invention is used for the treatment of Parkinson's disease or restless leg syndrome, no side effect due to an increase in the initial drug concentration in blood occurs and the skin penetration effect of the drug is excellent.

## Description

### Technical Field

The present invention relates to a transdermal absorption preparation.

More particularly, the present invention relates to a transdermal absorption preparation for treating a neurological disorder, which contains ropinirole or a salt thereof, which does not cause side effects attributable to an increase in the initial drug concentration in blood, and which has excellent performance of penetrating ropinirole or a salt thereof into the skin.

### Background Art

Ropinirole is selective to a D2 dopamine receptor, and has high affinity for a D3 dopamine receptor. Ropinirole enables dopamine to be easily transferred to a dopamine receptor.

When ropinirole is introduced into the body by oral administration as a drug delivery system, unfavorable side effects and inconveniences are caused. Typical examples of side effects may include swooning, narcolepsy, headache, nausea, fatigue, indigestion, and the like. It is determined that such side effects are caused by high initial blood drug concentration at the time of oral administration.

In order to solve the above problem, there is proposed a method of reducing the side effect attributable to the increase of the initial blood drug concentration by continuously introducing a predetermined amount of drug into the body using a transdermal absorption preparation including ropinirole through a drug delivery system.

In a transdermal administration system, unlike conventional transdermal administration or conventional drug injection, a drug does not pass through the liver, so that it is possible to prevent the drug from being converted into an inactive metabolite by the liver. Further, in the transdermal administration system, a drug does not pass through the digestive organs, so that the disorder of the digestive organs by the drug can be minimized, and the problem of the absorptivity of the drug being influenced by the conditions in the digestive organs doe not occur. Further, the transdermal administration system is advantageous in that it can maintain a blood drug concentration constant for a long period of time, can prevent the side effects caused by a high blood drug concentration compared to an oral agent or an injection, can reduce the number of administration of the drug, and can discontinue the application of a drug when side effects occur. Further, the transdermal administration system is advantageous in that it can be easily applied to patients who cannot swallow a drug.

However, regardless of such advantages, the transdermal administration system is problematic in that the skin acts as a barrier to the introduction of foreign matter into the body, so that the skin itself prevents a drug from permeating into the body, thereby restricting the permeability of a drug. Therefore, the kinds of drugs that can be introduced into the body through the skin are limited. Moreover, there is a problem in that hydrophilic drugs or salt-type drugs are difficult to be absorbed into the body through the skin compared to general drugs because they have high self-polarity.

In order to solve the above problems, various methods have been developed. Typically, there are methods of uniformly partitioning drug concentration from the transdermal administration system to the skin by increasing the concentration of drug in the transdermal administration system and methods of easily delivering a drug to the skin using ultrasonic waves, electric current or the like.

Meanwhile, unlike base materials used in an oral agent, an absorbent, a suppository, an ointment and the like, a transdermal absorption preparation requires several characteristics because a drug is administered through the skin. The base materials used in a transdermal absorption preparation must have adhesive properties sufficient to attach the transdermal absorption preparation to the skin for a long period of time, must not irritate the skin, and must not leave residues on the skin when the transdermal absorption preparation is removed from the skin.

### Disclosure

### Technical Problem

Accordingly, an object of the present invention is to provide a novel transdermal absorption preparation, from which a drug is easily absorbed into the skin, which has adhesive properties sufficient to be attached to the skin, which does not irate the skin, and which does not leave residues on the skin when it is removed from the skin.

### Technical Solution

In order to accomplish the above object, an aspect of the present invention provides a transdermal absorption preparation, including: a drug-containing adhesive layer; a drug-protective layer; and a release layer, wherein the drug-containing adhesive layer includes ropinirole or a salt thereof, rubber, an adhesion-imparting resin, an antioxidant and a transdermal absorption promoter.

Another aspect of the present invention provides a transdermal absorption preparation, including: a drug-containing adhesive layer; a drug-protective layer; and a release layer, wherein the drug-containing adhesive layer includes ropinirole or a salt thereof, an acrylic adhesive, an anti-crystallization agent and a transdermal absorption promoter.

### Advantageous Effects

When the transdermal absorption preparation of the present invention is used for the treatment of a neurological disorder, such as Parkinson's disease, restless leg syndrome or the like, no side effect attributable to the increase in the initial drug concentration in blood occurs, and the skin penetration effect of a drug is excellent.

Further, the transdermal absorption preparation of the present invention has adhesive properties sufficient to be attached to the skin, does not irate the skin, and does not leave residues on the skin when it is removed from the skin.

### Description of Drawings

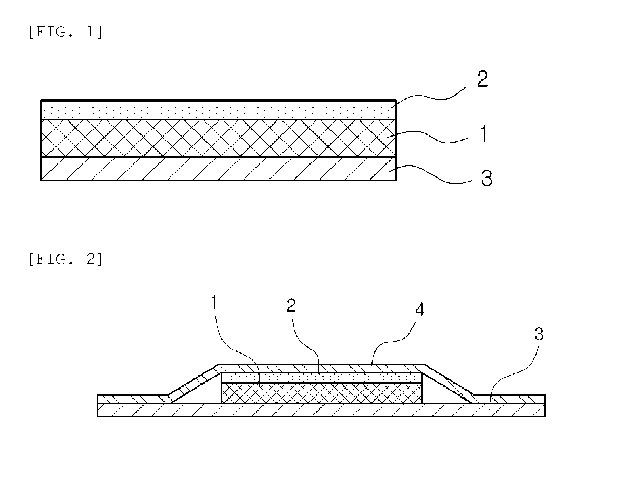

FIG. 1 is a sectional view showing a transdermal absorption preparation according to an embodiment of the present invention, in which 1 is a drug-containing adhesive layer, 2 is a drug-protective layer and 3 is a release layer.
FIG. 2 is a sectional view showing a transdermal absorption preparation according to another embodiment of the present invention, in which 1 is a drug-containing adhesive layer, 2 is a drug-protective layer, 3 is a release layer, and 4 is a support layer.

### Best Mode

The present invention provides a novel transdermal absorption preparation.

The transdermal absorption preparation according to an embodiment of the present invention includes: a drug-containing adhesive layer; a drug-protective layer; and a release layer, wherein the drug-containing adhesive layer includes ropinirole or a salt thereof, rubber, an adhesion-imparting resin, an antioxidant and a transdermal absorption promoter.

The drug-containing adhesive layer contains ropinirole or a salt thereof, and directly comes into contact with the skin to control a drug discharge rate. The drug-containing adhesive layer is a matrix-type single layer containing ropinirole or a salt thereof as a drug, and is directly brought into contact with the skin by a pressure-sensitive adhesion system after the release layer is taken off.

The thickness of the drug-containing adhesive layer may be 30 ~ 120 µm, and preferably, 50 ~ 80 µm. When the thickness thereof is less than 30 µm, a drug is crystallized in the transdermal absorption preparation because the concentration of the drug in the preparation becomes excessively high, and the adhesivity of the drug-containing adhesive layer becomes low because the concentration of an adhesive in the preparation becomes excessively low. Further, when the thickness thereof is more than 120 µm, the concentration of a drug in the preparation becomes low, so that it is difficult to allow a drug to permeate into the skin.

Ropinirole [4-(2-di-n-propylaminoethyl)-2-(3H)-indolone], which is an indolone derivative, is selective to a D2 dopamine receptor, and has high affinity for a D3 dopamine receptor. Ropinirole, represented by Chemical Formula 1 below, has a molecular weight of 260.38, and enables dopamine to be easily transferred to a dopamine receptor.

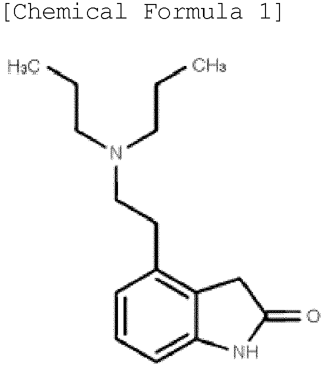

The salt of ropinirole may be a salt obtained from any one selected from the group consisting of inorganic acids, such as hydrochloric acid, nitric acid, sulfuric acid, hydrobromic acid, hydroiodic acid, nitrous acid, phosphorous acid and the like; and nonpoisonous organic acids, such as aliphatic monocarboxylic acids, aliphatic dicarboxylic acids, alkanoic acids substituted with a phenol group, hydroxyalkanoic acids, hydroxyalkanediol acids, aromatic acids, aliphatic sulfonic acids, aromatic sulfonic acids, and the like, but is not limited thereto.

The ropinirole or salt thereof may be included in an amount of 5 ~ 30 wt%, preferably, 10 - 20 wt% based on the total amount of the drug-containing adhesive layer. When the amount of the ropinirole or salt thereof is less than 5 wt%, there is a problem in that an optimum level of skin permeability cannot be accomplished. Further, when the amount of the ropinirole or salt thereof is more than 30 wt%, there is a problem in that drug concentration becomes high, thus crystallizing a drug and deteriorating the adhesivity of the drug-containing adhesive layer.

The rubber serves as an adhesive base material because it has a low glass transition temperature (Tg), high thermoplasticity and excellent mechanical properties. Preferably, since the adhesivity of the drug-containing adhesive layer is low when only the rubber is used, an adhesion-imparting resin may be added in order to obtain desired adhesive properties.

The rubber may be at least one selected from the group consisting of a styrene-ethylene-isoprene-styrene copolymer, a styrene-butadiene-styrene copolymer, a styrene-butadiene copolymer, a styrene-isoprene copolymer, a styrene-isoprene-styrene copolymer, silicon rubber, and polyisobutylene. This rubber can be used without limitation as long as it is a biocompatible rubber.

The rubber may be included in an amount of 10 - 50 wt% based on the total amount of the drug-containing adhesive layer. When the amount of the rubber is less than 10 wt%, there is a problem in that the mechanical properties of the drug-containing adhesive layer deteriorate because it cannot sufficiently serve as an adhesive base material. Further, when the amount thereof is more than 50 wt%, there is a problem in that the adhesivity of the drug-containing adhesive layer becomes low because the viscosity thereof becomes high at the time of compounding.

The adhesion-imparting resin is used to obtain desired adhesivity in a suitable amount when desired adhesivity cannot be obtained by only the rubber.

The adhesion-imparting resin may be at least one selected from the group consisting of a rogin glycerin ester resin, a hydrogenated rogin glycerin ester resin, a rogin pentaerythritol ester resin, a hydrogenated rogin pentaerythritol ester resin, an alpha-methyl styrene resin, an alicyclic saturated hydrocarbon resin of 5 to 9 carbon atoms, an aliphatic hydrocarbon resin of 5 to 9 carbon atoms, an aromatic hydrocarbon resin, a hydrogenated dicyclopentadiene resin, a terpene resin, a cumarone indene resin, and a terpene phenol resin. However, the present invention is not limited thereto.

The adhesion-imparting resin may be included in an amount of 5 ~ 70 wt% based on the total amount of the drug-containing adhesive layer. When the amount of the adhesion-imparting resin is less than 5 wt%, there is a problem in that the adhesivity of the transdermal absorption preparation is insufficiently improved. Further, when the amount thereof is more than 70 wt%, there is a problem in that the transdermal absorption preparation irritates the skin when it is taken off from the skin.

The antioxidant is used to prevent a polymer material constituting an adhesive layer from being cut or cross-linked by light or the reaction of the polymer material with oxygen to change the molecular structure and molecular weight thereof. Specific examples of the antioxidant may include phenol-based antioxidants, such as butylated hydroxytoluene (BHT), Irganox® 565 (manufactured by Chiba Geigy Corporation), Irganox 1010 (manufactured by Chiba Geigy Corporation), and Irganox 1076 (manufactured by Chiba Geigy Corporation). These phenol-based antioxidants act as a free radical remover, and are primary antioxidants which can be used independently or in combination with phosphite-based antioxidants. Further, these phenol-based antioxidants may be used independently or in combination with other antioxidants.

The antioxidant may be included in an amount of more than 0 wt% and 2 wt% or less, preferably, 0.1 ~ 1 wt% based on the total amount of the drug-containing adhesive layer. Since the antioxidant serves to prevent a polymer material from forming radicals using light or oxygen, the desired results can be obtained even when the antioxidant is used in a small amount.

The transdermal absorption promoter is used to increase the efficiency of delivering ropinirole or a salt thereof to the skin.

The transdermal absorption promoter may be at least one selected from the group consisting of polyoxyethylene monooleate, polyglyceryl diisostearate, N-methyl-2-pyrrolidone, N-carprylyl-2-pyrrolidone, N-dodecyl-2-pyrrolidone(lauryl pyrrolidone), lauryl alcohol, glycerol lauryl alcohol, oleyl alcohol, isopropyl myristrate, sorbitan monooleate, propylene monolaurate, propylene monooleate, oleoyl macrogolglyceride, oleic acid, lauroyl macrogolglyceride, linoleoyl macrogolglyceride, propylene glycol caprylate, propylene glycol caprate, sorbitan monostearate monooleate, glycerol monolaurate, glycerol monooleate, propylene glycol monolaurate, propylene glycol monocaprylate, sorbitan monolaurate, sorbitan monooleate, lauryl lactate, caprylic triglyceride, capric triglyceride, corn oil PEG-8 ester, corn oil PEG-6 ester, and triacetin. However, the present invention is not limited thereto.

The transdermal absorption promoter may be included in an amount of 5 - 20 wt% based on the total amount of the drug-containing adhesive layer. When the amount of the transdermal absorption promoter is less than 5 wt%, there is a problem in that the desired skin permeation promoting effect cannot be obtained. Further, when the amount thereof is more than 20 wt%, there is a problem in that adhesive properties deteriorate.

The drug-containing adhesive layer may further include a cohesion improver, such as silica dimethyl silylate or the like, in an amount of 0.1 ~ 10 wt% based on a total amount of the drug-containing adhesive layer. When the amount of the cohesion improver is less than 0.1 wt%, there is a problem in that the cohesion of the drug-containing adhesive layer is not sufficiently improved. Further, when the amount thereof is more than 10 wt%, there is a problem in that the adhesivity of the drug-containing adhesive layer deteriorates.

The drug-protective layer is used to prevent backside permeation of the drug included in the drug-containing adhesive layer. The thickness of the drug-protective layer is not particularly limited. However, it is preferred in terms of attachability or processability that the thickness thereof be 5 - 500 µm.

The drug-protective layer may be selected from the group consisting of a polyester film, a polypropylene film, a polyethylene film, a polyacrylonitrile film, a metal-deposited polyester film, and multilayered films thereof. The drug-protective layer may be a nonwoven fabric-laminated polyester film.

The release layer is removed and separated just before the transdermal absorption preparation is used, so that the drug-containing adhesive layer is directly attached to the skin. It is preferred in terms of detachability or processability that the thickness thereof be 15 - 500 µm.

The release layer may be formed by coating a polymer film made of any one selected from the group consisting of polyester, polyvinyl chloride, polyvinylidene chloride, polyethylene terephthalate and copolymers thereof with a silicon resin or a fluorine resin. Further, the release layer may be a release paper formed by coating a polyolefin-containing high-quality paper or a polyolefin-containing glassine paper with a silicon resin or a fluorine resin.

The transdermal absorption preparation may further include a support layer in order to increase the reliability of the preparation by preventing the preparation from being detached from the skin due to the excessive discharge of moisture or sweat and by maintaining the attachment of the preparation to the skin. It is preferred in terms of attachability or processability that the thickness of the support layer be 5 - 500 µm.

The support layer may be formed by applying any one selected from the group consisting of an acrylic adhesive, a rubber adhesive, a silicon adhesive and an ethylene-vinyl acetate (EVA) adhesive onto any one selected from the group consisting of a polyurethane film, a porous film, a perforated film, a woven cloth, a nonwoven cloth and a foam.

The transdermal absorption preparation according to another embodiment of the present invention includes: a drug-containing adhesive layer; a drug-protective layer; and a release layer, wherein the drug-containing adhesive layer includes ropinirole or a salt thereof, an acrylic adhesive, an anti-crystallization agent and a transdermal absorption promoter.

As described above, the ropinirole or salt thereof may be included in an amount of 5 - 30 wt%, preferably, 10 - 20 wt% based on the total amount of the drug-containing adhesive layer. When the amount of the ropinirole or salt thereof is less than 5 wt%, there is a problem in that the desired skin permeability cannot be accomplished. Further, when the amount of the ropinirole or salt thereof is more than 30 wt%, there is a problem in that drug concentration becomes high, thus crystallizing a drug and deteriorating the adhesivity of the drug-containing adhesive layer.

The acrylic adhesive may have no functional group or may have at least one functional group selected from the group consisting of - COOH, -OH and -NH₂. Specifically, the acrylic adhesive may be a polyacrylate block copolymer including at least one selected from the group consisting of acrylic acid, 2-ethylhexyl acrylate, butyl acrylate, 2-hydroxyethyl acrylate, methyl methacrylate, methyl acrylate, glycidyl methacrylate, vinyl acetate, N-vinyl-2-pyrrolidone, diacetone acrylamide, t-octyl acrylamide, and 2-(dimethyl aminoethyl) methacrylate.

The acrylic adhesive may be included in an amount of 30 - 90 wt%, preferably, 50 ~ 90 wt% based on the total amount of the drug-containing adhesive layer. When the amount of the acrylic adhesive is less than 30 wt%, there is a problem in that it is difficult to obtain the desired adhesive. Further, when the amount thereof is more than 90 wt%, there is a problem in that the concentration of a drug, a transdermal absorption promoter, or a release agent relatively decreases.

The anti-crystallization agent may be included in an amount of 0.1 ~ 20 wt% based on the total amount of the drug-containing adhesive layer. When the amount of the anti-crystallization agent is less than 0.1 wt%, there is a problem in that a crystallization prevention effect is insufficient. Further, when the amount thereof is more than 20 wt%, there is a problem in that the viscosity of a compounded solution becomes excessively high, and the adhesivity of the transdermal absorption preparation becomes low.

The anti-crystallization agent may be at least one selected from the group consisting of polyvinyl pyrrolidone, hydroxypropyl cellulose, hydroxypropyl methyl cellulose, and ethyl cellulose. Preferably, the anti-crystallization agent may be polyvinyl pyrrolidone. The anti-crystallization agent may serve as a release agent.

The transdermal absorption promoter may be at least one selected from the group consisting of polyoxyethylene monooleate, polyglyceryl diisostearate, N-methyl-2-pyrrolidone, N-carprylyl-2-pyrrolidone, N-dodecyl-2-pyrrolidone(lauryl pyrrolidone), lauryl alcohol, glycerol lauryl alcohol, oleyl alcohol, isopropyl myristrate, sorbitan monooleate, propylene monolaurate, propylene monooleate, oleoyl macrogolglyceride, oleic acid, lauroyl macrogolglyceride, linoleoyl macrogolglyceride, propylene glycol caprylate, propylene glycol caprate, sorbitan monostearate monooleate, glycerol monolaurate, glycerol monooleate, propylene glycol monolaurate, propylene glycol monocaprylate, sorbitan monolaurate, sorbitan monooleate, lauryl lactate, caprylic triglyceride, capric triglyceride, corn oil PEG-8 ester, corn oil PEG-6 ester, and triacetin. However, the present invention is not limited thereto.

The transdermal absorption promoter may be included in an amount of 5 - 20 wt% based on the total amount of the drug-containing adhesive layer. When the amount of the transdermal absorption promoter is less than 5 wt%, there is a problem in that the desired skin permeation promoting effect cannot be obtained. Further, when the amount thereof is more than 20 wt%, there is a problem in that adhesive properties deteriorate.

The drug-protective layer is used to prevent the backside permeation of the drug included in the drug-containing adhesive layer. The thickness of the drug-protective layer is not particularly limited. However, it is preferred in terms of attachability or processability that the thickness thereof be 5 - 500 µm.

The drug-protective layer may be selected from the group consisting of a polyester film, a polypropylene film, a polyethylene film, a polyacrylonitrile film, a metal-deposited polyester film, a nonwoven fabric-laminated polyester film, and multilayered films thereof.

The release layer is removed and separated just before the transdermal absorption preparation is used, so that the drug-containing adhesive layer is directly attached to the skin. It is preferred in terms of detachability or processability that the thickness thereof be 15 - 500 µm.

The release layer may be a polymer film made of any one selected from the group consisting of polyester, polyvinyl chloride, polyvinylidene chloride, polyethylene terephthalate and copolymers thereof. Further, the release layer may be a release paper formed by coating a polyolefin-containing high-quality paper or a polyolefin-containing glassine paper with a silicon resin or a fluorine resin. The transdermal absorption preparation may further include a support layer in order to increase the reliability of the preparation by preventing the preparation from being detached from the skin due to excessive discharge of moisture or sweat and by maintaining the attachment of the preparation to the skin. It is preferred in terms of attachability or processability that the thickness of the support layer be 5 - 500 µm.

The support layer may be formed by applying any one selected from the group consisting of an acrylic adhesive, a rubber adhesive, a silicon adhesive and an ethylene-vinyl acetate (EVA) adhesive onto any one selected from the group consisting of a polyurethane film, a porous film, a perforated film, a woven cloth, a nonwoven cloth and a foam.

The transdermal absorption preparation of the present invention is configured such that a drug can be continuously discharged for 1 ~ 7 days. Further, the transdermal absorption preparation is designed to have an area of 2.5 ~ 70 cm² according to the desired amount of a drug, and is configured such that the amount of a drug is changed according to the area thereof.

### Mode for Invention

Hereinafter, the present invention will be described in more detail with reference to the following Examples. However, the scope of the present invention is not limited to these Examples.

### <Comparative Example 1>

10 wt% of ropinirole, 12 wt% of a styrene-ethylene-isoprene-styrene copolymer, 12 wt% of a styrene-isoprene copolymer, 50.5 wt% of a hydrogenated alicyclic hydrocarbon (C₅) resin, 15 wt% of an alpha-methyl styrene resin and 0.5 wt% of butylated hydroxytoluene were accurately weighed, and were then completely dissolved in 175 g of toluene to prepare a solution.

Subsequently, the solution was applied onto a polyester film (thickness: 75 µm) coated with a silicon resin, and then dried to form a drug-containing adhesive layer having a thickness of 110 µm. Then, the drug-containing adhesive layer was laminated with a polyester film (thickness: 12 µm) to obtain a patch.

### <Comparative Example 2>

10 wt% of ropinirole, 55 wt% of polyisobutylene, 34.5 wt% of a hydrogenated rogin pentaerythritol ester resin and 0.5 wt% of butylated hydroxytoluene were accurately weighed, and were then completely dissolved in 175 g of toluene to prepare a solution.

Subsequently, the solution was applied onto a polyester film (thickness: 75 µm) coated with a silicon resin, and then dried to form a drug-containing adhesive layer having a thickness of 110 µm. Then, the drug-containing adhesive layer was laminated with a polyester film (thickness: 12 µm) to obtain a patch.

### <Examples 1 to 7>

The components of each of Examples 1 to 7, given in Table 1 below, were then completely dissolved in 175 g of toluene to prepare a solution. Subsequently, the solution was applied onto a polyester film (thickness: 75 µm) coated with a silicon resin, and then dried to form a drug-containing adhesive layer having a thickness of 110 µm. Then, the drug-containing adhesive layer was laminated with a polyester film (thickness: 12 µm) to obtain a patch.

### <Examples 8 to 14>

The components of each of Examples 8 to 14, given in Tables 1 and 2 below, were then completely dissolved in 175 g of toluene to prepare a solution. Subsequently, the solution was applied onto a polyester film (thickness: 75 µm) coated with a silicon resin, and then dried to form a drug-containing adhesive layer having a thickness of 73 µm. Then, the drug-containing adhesive layer was laminated with a polyester film (thickness: 12 µm) to obtain a patch.

**[Table 1]**

| Classification | | Example (wt%) | | | | | | | | | |
|---|---|---|---|---|---|---|---|---|---|---|---|
| | | 1 | 2 | 3 | 4 | 5 | 6 | 7 | 8 | 8 | 10 |
| Component | Ropinirole | 10 | 10 | 10 | 10 | 10 | 10 | 10 | 15 | 15 | 15 |
| | Styrene-ethylene-isoprene-styrene copolymer | 10 | 10 | 10 | 10 | 10 | | | 10 | 10 | 10 |
| | Styrene-isoprene copolymer | 10 | 10 | 10 | 10 | 10 | | | 10 | 10 | 10 |
| | Polyisobutylene | | | | | | 49 | 49 | | | |
| | Hydrogenated alicyclic hydrocarbon resin (C₅) | 46 | 46 | 46 | 46 | 46 | | | 38 | 38 | 38 |
| | Alpha-methyl styrene resin | 13.5 | 13.5 | 13.5 | 13.5 | 13.5 | | | 13.5 | 11.5 | 9.5 |
| | Hydrogenated rogin pentaerythritol ester | | | | | | 30.5 | 30.5 | | | |
| | Silica dimethyl silylate | | | | | | | | 3 | 3 | 3 |
| | Butylated hydroxytoluene | 0.5 | 0.5 | 0.5 | 0.5 | 0.5 | 0.5 | 0.5 | 0.5 | 0.5 | 0.5 |
| | Lauryl pyrrolidone | 10 | | | | | 10 | | 10 | 12 | 14 |
| | Isopropyl myristrate | | 10 | | | | | 10 | | | |
| | Glycerol monooleate | | | 10 | | | | | | | |
| | Propyleneglycol monolaurate | | | | 10 | | | | | | |
| | Triacetin | | | | | 10 | | | | | |

**[Table 2]**

| Classification | | Example (wt%) | | | | Comparative Example (wt%) | |
|---|---|---|---|---|---|---|---|
| | | 11 | 12 | 13 | 14 | 1 | 2 |
| Component | Ropinirole | 15 | 15 | 15 | 15 | 10 | 10 |
| | Styrene-ethylene-isoprene-styrene copolymer | 14 | 10 | 10 | 10 | 12 | |
| | Styrene-isoprene copolymer | 6 | 10 | 10 | 10 | 12 | |
| | Polyisobutylene | | | | | | 55 |
| | Hydrogenated alicyclic hydrocarbon resin (C₅) | 38 | 37 | | | 50.5 | |
| | Alpha-methyl styrene resin | 13.5 | 12.5 | 13.5 | 51.5 | 15 | |
| | Aliphatic hydrocarbon resin (C₅) | | | 38 | | | |
| | Hydrogenated rogin pentaerythritol ester | | | | | | 34.5 |
| | Silica dimethyl silylate | 3 | 5 | 3 | 3 | | |
| | Butylated hydroxytoluene | 0.5 | 0.5 | 0.5 | 0.5 | 0.5 | 0.5 |
| | Lauryl pyrrolidone | 10 | 10 | 10 | 10 | | |

### <Test Example (skin permeation test)>

The skin permeation test of each of the patches obtained in Examples 1 to 14 and Comparative Examples 1 and 2 was carried out using a Franz diffusion cell (effective area: 0.64 cm², aqueous solution volume: 5.2 mℓ) under a sink condition. A phosphate buffered saline solution (PBS) having a pH of 7.4 was used as an aqueous solution.

First, a Franz diffusion cell was filled with an aqueous solution, and was then maintained at 32±0.5°C. Subsequently, a sample was cut in the shape of a circle (area: 0.64cm²), and was then attached to the center of a skin sample (human cadaver skin epidermis). Then, the skin sample provided with the sample was disposed on an acceptor of the Franz diffusion cell, and was covered with a donor and fixed using a clamp. Thereafter, the skin permeation test thereof was carried out.

As the skin sample, human cadaver skin epidermis was used. The human cadaver skin epidermis was stored at -70°C. The human cadaver skin epidermis was unfrozen at 40°C and then used. The analysis of skin permeation was performed using HPLC under the conditions that a 70 mM ammonium formate solution (pH: 3.8) including acetonitrile, 0.3% of EDTA and 0.005% of sodium octyl sulfate at a volume ratio of 25:75 was used as a moving fluid, the amount of the moving fluid was 20 µℓ, the flow rate thereof was 0.8 mℓ/min, detection wavelength was 250nm, and a C18 reverse phase column was used as a column.

The results thereof measured for 24 hours are given in Table 3 below, and the results thereof measured for 48 hours (Examples 9 and 10) are given in Table 4 below.

**[Table 3]**

| Skin permeation test results (human cadaver skin): accumulated skin permeation amount and skin permeation rate for 24 hours | | | | | |
|---|---|---|---|---|---|
| Class. | Accumulated skin permeation amount (µg/cm²) | Skin permeation rate (µg/cm²/hr) | Class. | Accumulated skin permeation amount (µg/cm²) | Skin permeation rate (µg/cm²/hr) |
| Comp. Ex. 1 | 24.0±2.7 | 1.0 | Ex. 7 | 100.3±3.5 | 4.2 |
| Comp. Ex. 2 | 16.3±4.6 | 0.7 | Ex. 8 | 289.7±15.0 | 12.1 |
| Ex. 1 | 146.3±8.3 | 6.1 | Ex. 9 | 331.0±17.2 | 13.8 |
| Ex. 2 | 144.1±10.3 | 6.0 | Ex. 10 | 367.8±21.2 | 15.3 |
| Ex. 3 | 110.7±3.7 | 4.6 | Ex. 11 | 298.5±16.5 | 12.4 |
| Ex. 4 | 75.6±2.7 | 3.2 | Ex. 12 | 278.3±22.6 | 11.6 |
| Ex. 5 | 72.2±1.9 | 3.0 | Ex. 13 | 238.7±12.3 | 9.9 |
| Ex. 6 | 103.7±5.3 | 4.3 | Ex. 14 | 285.7±18.1 | 11.9 |

**[Table 4]**

| Skin permeation test results (human cadaver skin): accumulated skin permeation amount and skin permeation rate for 48 hours | | |
|---|---|---|
| Class. | Accumulated skin permeation amount (µg/cm²) | Skin permeation rate (µg/cm²/hr) |
| Ex. 9 | 514.0±20.3 | 10.7 |
| Ex. 10 | 642.2±30.6 | 13.4 |

### <Comparative Examples 3 to 6 and Examples 15 to 22>

The two components (ropinirole and polyvinylpyrrolidone) of each of Comparative Examples 3 to 6 and Examples 15 to 22, given in Table 5 below, were completely dissolved in 10 g of isopropyl alcohol and 57.4 g of toluene to prepare a first solution. Subsequently, the first solution was mixed with other components (each of Duro-tak® 387-2287, Duro-tak® 87-2852, Duro-tak® 87-4098, and SK-Dyne® IC-01N (AM) is used as an adhesive and is a solution phase in which solids are dissolved in ethyl acetate or the like, and the content ratios given in Table 5 below are based on solid content) in each of the content ratios given in Table 5 below to prepare a second solution. Subsequently, the second solution was applied onto a polyester film (thickness: 75 µm) coated with a silicon resin, and was then dried to form a drug-containing adhesive layer having a thickness of 66 µm. Then, the drug-containing adhesive layer was laminated with a polyester film (thickness: 12 µm) to obtain a patch.

**[Table 5]**

| Class. | | Example (wt%) | | | | | | | | Comparative Example (wt%) | | | |
|---|---|---|---|---|---|---|---|---|---|---|---|---|---|
| | | 15 | 16 | 17 | 18 | 19 | 20 | 21 | 22 | 3 | 4 | 5 | 6 |
| Component | Ropinirole | 15 | 15 | 15 | 15 | 15 | 15 | 15 | 15 | 15 | 15 | 15 | 15 |
| | Polyvinyl pyrrolidone | 0.5 | 0.5 | 0.5 | 0.5 | 0.5 | 0.5 | 0.5 | 0.5 | 0.2 | 0.2 | 0.2 | 0.2 |
| | Lauryl pyrrolidone | 10 | | | | | | | | | | | |
| | Lauryl alcohol | | 10 | | | | | | | | | | |
| | Propyleneglycol monolaurate | | | 10 | | | | | | | | | |
| | Polyoxyethylene monooleate | | | | 10 | | | | | | | | |
| | Oleoyl macrogolglyceride | | | | | 10 | | | | | | | |
| | Polyglyceryl diisostearate | | | | | | 10 | | | | | | |
| | Sobitan monooleate | | | | | | | 10 | | | | | |
| | glycerol monooleate | | | | | | | | 10 | | | | |
| | Duro-tak® 387- | 74. | 74. | 74. | 74. | 74. | 74. | 74. | 74. | 84. | | | |
| | 2287 | 5 | 5 | 5 | 5 | 5 | 5 | 5 | 5 | 8 | | | |
| | Duro-tak® 87-2852 | | | | | | | | | | 84. 8 | | |
| | SK-Dyne® IC-01N (AM) | | | | | | | | | | | 84. 8 | |
| | Duro-tak® 87-4098 | | | | | | | | | | | | 84. 8 |

Duro-tak® 387-2287, Duro-tak® 87-2852, and Duro-tak® 87-4098, each of which was used as the adhesive component, were manufactured by National Starch Chemical Corporation (Bridgewater, N.J., USA), and SK-Dyne® IC-01N (AM) was manufactured by Soken Chemical & Engineering Corporation (Takada, Toshima-ku, Tokyo, Japan).

The skin permeation test of each of the patches obtained in Examples 15 to 22 and Comparative Examples 3 to 6 was carried out in the same manner as in each of Examples 1 to 14 and Comparative Examples 1 and 2. The results thereof are given in Table 6 below.

**[Table 6]**

| Skin permeation test results (human cadaver skin): accumulated skin permeation amount and skin permeation rate for 24 hours | | | | | |
|---|---|---|---|---|---|
| Class. | Accumulated skin permeation amount (µg/cm²) | Skin permeation rate (µg/cm²/hr) | Class. | Accumulated skin permeation amount (µg/cm²) | Skin permeation rate (µg/cm²/hr) |
| Ex. 15 | 381.5±48.1 | 15.9 | Ex. 21 | 196.5±11.0 | 8.2 |
| Ex. 16 | 287.7±22.4 | 12.0 | Ex. 22 | 203.8±10.6 | 8.5 |
| Ex. 17 | 300.2±56.5 | 12.5 | Comp. Ex. 3 | 188.4±32.4 | 7.9 |
| Ex. 18 | 223.6±4.7 | 9.3 | Comp. Ex. 4 | 17.0±8.4 | 0.7 |
| Ex. 19 | 244.8±54.8 | 10.2 | Comp. Ex. 5 | 107.5±10.5 | 4.5 |
| Ex. 20 | 199.2±3.4 | 8.3 | Comp. Ex. 6 | 168.3±19.2 | 7.0 |

Referring to Table 3 and Table 6 above, it can be ascertained that the skin permeation effects of the transdermal absorption preparations of Examples are excellent compared to those of the transdermal absorption preparations of Comparative Examples.

Although the preferred embodiments of the present invention have been disclosed for illustrative purposes, those skilled in the art will appreciate that various modifications, additions, and substitutions are possible, without departing from the scope and spirit of the invention as disclosed in the accompanying claims.

## Claims

1. A transdermal absorption preparation, comprising:
a drug-containing adhesive layer;
a drug-protective layer; and
a release layer, wherein the drug-containing adhesive layer includes ropinirole or a salt thereof, rubber, an adhesion-imparting resin, an antioxidant and a transdermal absorption promoter.

2. A transdermal absorption preparation, comprising:
a drug-containing adhesive layer;
a drug-protective layer; and
a release layer, wherein the drug-containing adhesive layer includes ropinirole or a salt thereof, an acrylic adhesive, an anti-crystallization agent and a transdermal absorption promoter.

3. The transdermal absorption preparation of claim 1 or 2, further comprising a support layer.

4. The transdermal absorption preparation of claim 1 or 2, wherein the ropinirole or the salt thereof is included in an amount of 5 - 30 wt% based on a total amount of the drug-containing adhesive layer.

5. The transdermal absorption preparation of claim 1, wherein the rubber is at least one selected from the group consisting of a styrene-ethylene-isoprene-styrene copolymer, a styrene-butadiene-styrene copolymer, a styrene-butadiene copolymer, a styrene-isoprene copolymer, a styrene-isoprene-styrene copolymer, silicon rubber, and polyisobutylene.

6. The transdermal absorption preparation of claim 1, wherein the rubber is included in an amount of 10 - 50 wt% based on a total amount of the drug-containing adhesive layer.

7. The transdermal absorption preparation of claim 1, wherein the adhesion-imparting resin is at least one selected from the group consisting of a rogin glycerin ester resin, a hydrogenated rogin glycerin ester resin, a rogin pentaerythritol ester resin, a hydrogenated rogin pentaerythritol ester resin, an alpha-methyl styrene resin, an alicyclic saturated hydrocarbon resin of 5 to 9 carbon atoms, an aliphatic hydrocarbon resin of 5 to 9 carbon atoms, an aromatic hydrocarbon resin, a hydrogenated dicyclopentadiene resin, a terpene resin, a cumarone indene resin, and a terpene phenol resin.

8. The transdermal absorption preparation of claim 1, wherein the adhesion-imparting resin is included in an amount of 5 - 70 wt% based on a total amount of the drug-containing adhesive layer.

9. The transdermal absorption preparation of claim 1, wherein the antioxidant is any one selected from the group consisting of butylated hydroxytoluene (BHT), Irganox 565, Irganox 1010, Irganox 1076, phosphite, and mixtures thereof.

10. The transdermal absorption preparation of claim 1, wherein the antioxidant is included in an amount of more than 0% and 2 wt% or less based on a total amount of the drug-containing adhesive layer.

11. The transdermal absorption preparation of claim 1 or 2, wherein the transdermal absorption promoter is at least one selected from the group consisting of polyoxyethylene monooleate, polyglyceryl diisostearate, N-methyl-2-pyrrolidone, N-carprylyl-2-pyrrolidone, N-dodecyl-2-pyrrolidone(lauryl pyrrolidone), lauryl alcohol, glycerol lauryl alcohol, oleyl alcohol, isopropyl myristrate, sorbitan monooleate, propylene monolaurate, propylene monooleate, oleoyl macrogolglyceride, oleic acid, lauroyl macrogolglyceride, linoleoyl macrogolglyceride, propylene glycol caprylate, propylene glycol caprate, sorbitan monostearate monooleate, glycerol monolaurate, glycerol monooleate, propylene glycol monolaurate, propylene glycol monocaprylate, sorbitan monolaurate, sorbitan monooleate, lauryl lactate, caprylic triglyceride, capric triglyceride, corn oil PEG-8 ester, corn oil PEG-6 ester, and triacetin.

12. The transdermal absorption preparation of claim 1 or 2, wherein the transdermal absorption promoter is included in an amount of 5 - 20 wt% based on a total amount of the drug-containing adhesive layer.

13. The transdermal absorption preparation of claim 2, wherein the acrylic adhesive has no functional group or has at least one functional group selected from the group consisting of -COOH, -OH and -NH₂.

14. The transdermal absorption preparation of claim 2, wherein the acrylic adhesive is included in an amount of 30 - 90 wt% based on a total amount of the drug-containing adhesive layer.

15. The transdermal absorption preparation of claim 2, wherein the anti-crystallization agent is at least one selected from the group consisting of polyvinyl pyrrolidone, hydroxypropyl cellulose, hydroxypropyl methyl cellulose, and ethyl cellulose.

16. The transdermal absorption preparation of claim 2, wherein the anti-crystallization agent is included in an amount of 0.1 ~ 20 wt% based on a total amount of the drug-containing adhesive layer.

17. The transdermal absorption preparation of claim 1 or 2, wherein the drug-protective layer is selected from the group consisting of a polyester film, a polypropylene film, a polyethylene film, a polyacrylonitrile film, a metal-deposited polyester film, a nonwoven fabric-laminated polyester film, and multilayered films thereof.

18. The transdermal absorption preparation of claim 1 or 2, wherein the release layer is formed by coating a polymer film made of any one selected from the group consisting of polyester, polyvinyl chloride, polyvinylidene chloride, polyethylene terephthalate and copolymers thereof with a silicon resin or a fluorine resin, or is a release paper formed by coating a polyolefin-containing high-quality paper or a polyolefin-containing glassine paper with a silicon resin or a fluorine resin.

19. The transdermal absorption preparation of claim 3, wherein the support layer is formed by applying any one selected from the group consisting of an acrylic adhesive, a rubber adhesive, a silicon adhesive and an ethylene-vinyl acetate (EVA) adhesive onto any one selected from the group consisting of a polyurethane film, a porous film, a perforated film, a woven cloth, a nonwoven cloth and a foam.

20. The transdermal absorption preparation of claim 1, wherein the drug-containing adhesive layer further includes a cohesion improver in an amount of 0.1 ~ 10 wt% based on a total amount of the drug-containing adhesive layer.

21. The transdermal absorption preparation of claim 20, wherein the cohesion improver is silica dimethyl silylate.

22. The transdermal absorption preparation of claim 1 or 2, wherein the drug-containing adhesive layer has a thickness of 30 ~ 120 µm.

23. The transdermal absorption preparation of claim 1 or 2, wherein the drug-protective layer has a thickness of 5 - 500 µm.

24. The transdermal absorption preparation of claim 1 or 2, wherein the release layer has a thickness of 15 - 500 µm.

25. The transdermal absorption preparation of claim 3, wherein the support layer has a thickness of 5 - 500 µm.

26. The transdermal absorption preparation of claim 1 or 2, wherein the permeation rate of the ropinirole or the salt thereof is 3 - 200 µg/cm²/hr when the ropinirole or the salt thereof is absorbed in the skin at a predetermined absorption rate for 1 - 7 days.
